# EUROPEAN PATENT SPECIFICATION

(11) **EP 0 457 334 B1**
(45) Date of publication and mention of the grant of the patent: **10.08.1994**
(21) Application number: 91107976.2
(22) Date of filing: 16.05.1991
(51) Int. Cl.: C07C 69/96, C07C 317/22, C07C 245/08, C07C 291/08, C07C 251/24, C07C 323/20

(54) **Carbonates of acetylenic alcohols**
Carbonate von acetylenischen Alkoholen
Carbonates d'alcools acétyléniques

(30) Priority: 17.05.1990 JP 127783/90; 17.05.1990 JP 127785/90
(43) Date of publication of application: 21.11.1991
(73) Proprietor: NIPPON PAINT CO., LTD., Osaka-shi Osaka 572 (JP)
(72) Inventor: Yamada, Mitsuo, Suita, Osaka-fu (JP); Aoki, Kei, Ikoma, Nara-ken (JP); Urano, Satoshi, Tuszuki-gun, Kyoto-fu (JP); Mizuguchi, Ryuzo, Yawata, Kyoto-fu (JP)
(74) Representative: Kinzebach, Werner, Dr.

(56) References cited:
- FR-A- 2 212 178
- US-A- 3 120 556
- US-A- 3 134 802

## Description

This invention relates to novel carbonates of acetylenic alcohols.

It has been known that certain compounds having acetylenically unsaturated groups such as ethynyl or propynyl group may be polymerized into a conjugated diene polymer. Because of their unique polymerization mechanism and the unique electrical and physical properties of their polymers, such compounds are attracting a great interest as a component for producing self-curable, nonemanating resinous compositions. Japanese Patent Kokai No. 108213/89, for example, discloses a propargyl ether of cresol novolac resin as one of such compounds.

The present invention provides a novel class of compounds in which an acetylenic alcohol moity is attached to a phenolic moiety via a carbonate linkage to improve the heat resistance of the resultant polymers.

### Summary of the Invention

According to the present invention, there is provided a compound of the formula: 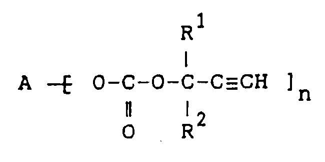
wherein A is an aromatic polycyclic system selected from the group consisting of a fused carbocycle having more than one benzene rings arranged in a straight row, a system having two benzene rings linked together with a direct bond, a C₁-C₂₀ alkylidene group, a cyclohexylidene group, -O-, -S-, -CO-, 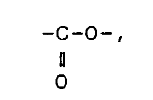
-CH=N-, 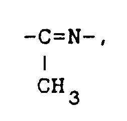
-N=N-, -SO₂- or 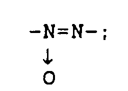
and derivatives thereof having on their benzene ring up to four substituents selectected from the group consisting of a C₁-C₁₂ alkyl, a C₁-C₁₂ alkoxy, halo, nitro and cyano;
R¹ and R² are independently a hydrogen atom or C₁-C₁₂ alkyl; and
n is an integer of 1, 2 or 3.

The compound of this invention may be synthesized by reacting a phenol of the formula:

A ⁅ OH ]ₙ

wherein A and n are as defined, with a chloroformate of the formula: 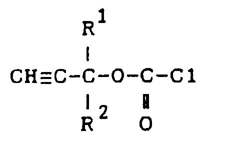
wherein R¹ and R² are as defined, in the presence of an acid acceptor, or alternatively reacting a chloroformate of the formula: 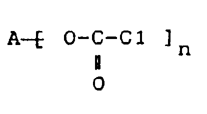
wherein A and n are as defined, with an alcohol of the formula: 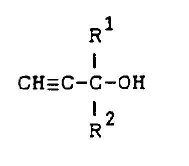
wherein R¹ and R² are as defined, in the presence of an acid acceptor.

### Detailed Discussion

The compounds of the invention are carbonate esters of an acetylenic alcohol such as propargyl alcohol and a mono- di or triphenol. Such carbonate esters may be synthesized, as is well-known, by reacting phosgene with either one of the phenol or the acetylenic alcohol to produce the corresponding chloroformate followed by the reaction of the latter with the other in the presence of an acid accepter.

As one class of the starting phenols, the present invention employs a polycyclic phenol capable of providing molecular orientating, liquid crystalline and optically anisotropic properties to the carbonate polymers. It has been known that such properties may be given by incorporating into the polymer chain a plurality of biphenyl groups or a fused carbocycle system having more than one benzene ring in a linear arrangement such as naphthalene, anthracene and naphthacene. Phenols of this class are those derived from biphenyl or the fused carbocycle system, or the corresponding phenols having up to four substituents selected from the group consisting of a C₁-C₁₂ alkyl, a C₁-C₁₂ alkyoxy, halo, nitro and cyano.

Specific examples of phenols of this class include:
O-phenylphenol,
m-phenylphenol,
p-phenylphenol,
4,4-hydroxybiphenyl,
2,2′-hydroxybiphenyl,
3-chloro-4,4′-dihydroxybiphenyl,
α-naphthol,
β-naphtol,
2,6-dihydroxynaphthalene,
2,7-dihydroxynaphthalene,
2-hydroxyanthracene,
9-hydroxyanthracene,
1,2-dihydroxyanthracene,
1,4-dihydroxyanthracene,
1,5-dihydroxyanthracene,
1,8-dihydroxyanthracene,
2,3-dihydroxyanthracene,
2,6-dihydroxyanthracene,
2,7-dihydroxyanthracene,
1,9-dihydroxyanthracene, and
9,10-dihydroxyanthracene.

Another class of starting phenols used in the present invention include bisphenols in which two benzene rings are linked through a bridge selected from the group consisting of a C₁-C₂₀ alkylidene group, a cyclohexylidene group, -O-, -S-, -CO-, -SO₂- 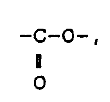
-CH=N-, 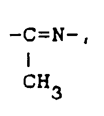
-N=N- and 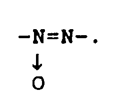
Again the bisphenols may have on the benzene ring up to four substituents selected from the group consisting of a C₁-C₁₂ alkyl, a C₁-C₁₂ alkoxy, halo, nitro and cyano.

Specific examples of bisphenols include:
bis(4-hydroxylphenyl)methane;
bis(4-hydroxy-2-methylphenyl)methane;
1,1-bis(4-hydroxyphenyl)ethane;
1,1-bis(4-hydroxy-3-methyl-5-t-butylphenyl)butane;
bis(4-hydroxy-2-chlorophenyl)methane;
bis(4-hydroxyphenyl)tridecane;
bis(2-hydroxyphenyl)methane;
bis(3-hydroxyphenyl)methane;
bis(2-hydroxy-4-methylphenyl)methane;
bis(2-hydroxy-5-methylphenyl)methane;
bis(2-hydroxy-6-methylphenyl)methane;
bis(4-hydroxy-3-methylphenyl)methane;
bis(3-hydroxy-2,4,6-trimethylphenyl)methane;
bis(2-hydroxy-4-propylphenyl)methane;
bis(4-hydroxy-2-propylphenyl)methane;
bis(4-hydroxy-2-methyl-6-ethylphenyl)methane;
bis(4-hydroxy-2,3,5,6-tetramethylphenyl)methane;
bis(2-hydroxy-4-t-butylphenyl)methane;
bis(4-hydroxy-2-methyl-5-isopropylphenyl)methane;
bis(4-hydroxy-2-t-butyl-5-methylphenyl)methane;
bis(4-hydroxy-2,6-di-t-butylphenyl)methane;
bis(4-hydroxy-2,6-dichloropheyl)methane;
bis(2-hydroxy-4-bromophenyl)methane;
bis(3-hydroxy-2,4,6-trichlorophenyl)methane;
bis(4-hydroxy-2-methoxyphenyl)methane;
1,1-bis(4-hydroxyphenyl)cyclohexane;
2,2′-thiobisphenol;
4,4′-thiobisphenol;
4,4′-thiobis(2-methylphenol);
2,2′-thiobis(4,5-dimethylphenol);
2,2′-thiobis(4,6-dimethylphenol);
4,4′-thibis(2,6-dimethylphenol);
2,2′-thiobis(6-t-butyl-4-methylphenol);
4,4′-thiobis(2-t-butyl-5-methylphenol);
2,2′-thiobis(4-fluorophenol);
2,2′-thiobis(4-chlorophenol);
4,4′-thiobis(3-chlorophenol);
2,2′-thiobis(4,6-dichlorophenol);
4,4′-thiobis(2-bromophenol);
2,2′-thiobis(5-nitrophenol);
bis(4-hydroxyphenyl)sulfone;
bis(2-hydroxyphenyl)sulfone;
bis(4-hydroxy-2-methylphenyl)sulfone;
bis(4-hydroxy-2,5-dimethylphenyl)sulfone;
bis(4-hydroxy-2-t-butyl-5-methylphenyl)sulfone;
bis(4-hydroxy-2-chlorophenyl)sulfone;
bis(4-hydroxy-3-chlorophenyl)sulfone;
bis(4-hydroxy-2-bromophenyl)sulfone;
bis(4-hydroxy-2-nitrophenyl)sulfone;
4,4′-dihydroxybenzophenone;
2,2′-dihydroxy-4-methoxybenzophenone;
4,4′-dihydroxydiphenyl ether;
4,4′-dihydroxyphenylbenzoate;
4,4′-dihydroxyazobenzene;
4,4′-dihydroxyazoxybenzene;
4′-hydroxybenzylidene-4′-hydroxyaniline;
4′-hydroxy-α-methylbenzylidene-4-hydroxyaniline;
4,4-dihydroxydiphenyl sulfide; and the like.

Acetylenic alcohols used in the synthesis of the carbonates of this inventin include propargyl alcohol and its α-mono- and α,α-di-C₁-C₂₀ alkyl derivatives. Propargyl alcohol is preferred.

The carbonate compounds of this invention are synthesized by reacting either one of the phenol component and the acetylenic alcohol component with phosgene to form the corresponding chloroformate following by reacting the latter with the other component. The reaction of the chloroformate of acetylenic alcohol with the phenol is preferred.

The reaction may be performed, as is conventional, in an inert solvent in the presence of an acid acceptor.

Examples of usable inert solvents include DMSO, DMF, N-methylpyrrolidone, acetone, methyl ethyl ketone, methyl isobutyl ketone, benzene, toluene, xylene, diethyl ether, tetrahydrofuran, dioxane, methylene chloride, dichloroethane and the like. When the reaction system is not too viscous, the reaction may be performed without using the solvent.

Examples of acid acceptors include trimethylamine, triethylamine, pyridine, sodium hydroxide, potassium hydroxide, calcium hydroxide, magnesium hydroxide, sodium silicate, sodium aluminate, sodium carbonate, potassium carbonate, alkali metal ethoxide and the like. Tertiary amines and pyridine are preferred.

The reaction is preferably carried out under the nitrogen gas atmosphere to prevent coloration of the product. The reaction temperature may range from room temperature to the boiling point of the solvent used.

After the reaction, the product may be isolated and purified by the conventional technique such as extraction, recrystallization and the like.

The carbonate compounds thus prepared may be polymerized with a metallic catalyst or initiator, or by irradiating with actinic radiations such as UV radiation, gamma radiation or electron beam radiation. They are, therefore, useful as stock materials of resins used in paints, electric and electronic components, structural materials and nonlinear optical materials where nonemanating cure, and durability and heat restance properties are desired in the finished products.

The invention is illustrated by the following examples wherein all parts and percents are by weight unless otherwise specified.

### Example 1

### Bis(4-propargyloxycarbonyloxyphenyl)methane

A flask equipped with a stirrer, thermoter, nitrogen gas tube and reflux condenser was charged with 20.0 g of bis(4-hydroxyphenyl)methane, 50.0 g of methylene chloride and 23.0 g of trimethylamine. To the flask was added 26.0 g of propargyl chloroformate dropwise over one hour and the mixture was allowed to react for 6 hours at 30 °C. After the reaction, the reaction mixture was treated with methylene chloride-water mixture and the organic phase was separated followed by drying over magnesium sulfate overnight. After filtering, the filtrate was evaporated in a rotary evaporator. The resulting crystals were recrystallized to give the title compound melting at 60 °C at a yield of 93.6 % of theory.

The structure of the product was identified by the IR spectrum and ¹H-NMR (in CDC1₃, trimethylsilane standard).
IR : 2980 cm⁻¹ (-CH₂-) ; 3300 cm⁻¹,
2100 cm⁻¹ (CH≡C-) ; 1738 cm⁻¹ (-OCOO-) ;
1600 cm⁻¹, 1500 cm⁻¹ (Phe) ;
1450 cm⁻¹ (-CH₂-)
¹H-NMR : δ (ppm)
2.41 (CH≡C-) ; 4.87 (-CH₂-) ; 6.96,
7.15 (Phe) ; 2.54 (-CH₂-)

### Example 2

### Bis(4-proparagyloxycarbonyloxy-2-methylphenyl)methane

The title compound was produced by reacting 23.0 g of bis(4-hydroxy-2-methylphenyl)methane and 26.0 g of propargyl chloroformate in 50.0 g of methylene chloride in the presence of 23.0 g of trimethylamine as in Example 1. Yield was 83.6 % of theory.
IR : 2990 cm⁻¹ (-CH₃) ; 3320 cm⁻¹,
2100 cm⁻¹ (CH≡C-) ; 1740 cm⁻¹ (-OCOO-) ;
1600 cm⁻¹, 1500 cm⁻¹ (Phe)
1450 cm⁻¹ (-CH₃)
¹H-NMR : δ (ppm)
2.42 (CH≡C-) ; 4.89 (-CH₂-) ; 6.75,
7.13 (Phe) ; 2.64 (-CH₂-) ;
1.64 (-CH₃)

### Example 3

### Bis(4-propargyloxycarbonyloxyphenyl)sulfone

The title compound was produced by reacting 20.0 g of bis(4-hydroxyphenyl)sulfone and 26.6 g of propargyl chloroformate in 50.0 g of methylene chloride in the presence of 23.0 g of trimethylamine as in Example 1. m.p.112-114 °C. Yield was 73.6 % of theory.
IR (cm⁻¹) : 3300, 2110 (CH≡C-) ;
1738 (-OCOO-) ; 1600, 1500 (Phe) ; 1350 (-SO₂-)
¹H-NMR : δ (ppm)
2.55 (CH≡C-) ; 4.86 (-CH₂-) ;
7.30, 7.90 (Phe)

### Example 4

### 4,4′-Bis(propargyloxycarbonyloxy)benzophenone

The title compound was produced by reacting 23.0 g of 4,4′-dihydroxybenzophenone and 26.0 g of propargyl chloroformate in 50.0 g of methylene chloride in the presence of 23.0 g of trimethylamine as in Example 1. m.p.104-106 °C. Yield was 83.6 % of theory.
IR (cm⁻¹) : 3300, 2100 (CH≡C-) ;
1755 (-OCOO-) ; 1610, 1510 (Phe)
¹H-NMR, δ (ppm) :
2.42 (CH≡C-) ; 4.89 (-CH₂-) ;
7.30, 7.90 (Phe)

Analogous to Example 1, following compound were produced

### Example 5

### 1,1-Bis(4-propargyloxycarbonyloxyphenyl)cyclohexane

IR (cm⁻¹) :
3300, 2100 (CH≡C-) ; 1740 (-OCOO-) ;
1610, 1500 (Phe) ;
2920, 1450 (-CH₂-)
¹H-NMR, δ (ppm) :
2.42 (CH≡C-) ; 4.89 (-CH₂-) ; 6.90,
7.12 (Phe)

### Example 6

### 1,1-Bis(4-propargyloxycarbonyloxyphenyl)ethane

IR (cm⁻¹) :
3300, 2100 (CH≡C-) ; 1750 (-OCOO-) ;
1610, 1500 (Phe) ;
2980 (-CH₃)
¹H-NMR, δ (ppm) :
1.11 (-CH₃) ; 2.40 (CH≡C-) ;
4.90 (-CH₂-) ; 6.90, 7.22 (Phe)

### Example 7

### 1,1-Bis(4-propargyloxycarbonyloxy-3-butylphenyl)butane

IR (cm⁻¹) :
3300, 2100 (CH≡C-) ; 1750 (-OCOO-) ;
1610, 1500 (Phe) ;
2980 (-CH₃) ; 1240 (t-butyl)
¹H-NMR, δ (ppm) :
1.11 (-CH₃) ; 2.40 (CH≡CO-) ;
4.90 (-CH₂-) ; 6.85, 7.12 (Phe)

### Example 8

### Bis(2-chloro-4-propargyloxycarbonyloxyphenyl)methane

IR (cm⁻¹) :
3300, 2100 (CH≡C-) ; 1750 (-OCOO-) ;
1600, 1500 (Phe)
1100 (Cl)
¹H-NMR, δ (ppm) :
2.42 (CH≡C-) ; 2.60 (-CH₂-) ;
4.88 (≡C-CH₂-) ; 6.78, 7.10 (Phe)

### Example 9

### 1,1-Bis(4-propargyloxycarbonyloxyphenyl)tridecane

IR (cm⁻¹) :
3300, 2110 (CH≡C-) ; 1750 (-OCOO-) ;
1610, 1500 (Phe)
¹H-NMR, δ (ppm) :
2.40 (CH≡C-) ; 2.58 (-CH₂-) ;
4.90 (≡C-CH₂-) ; 6.90, 7.22 (Phe)

### Example 10

### 4,4′-Bis(propargyloxycarbonyloxy)diphenyl ether

IR (cm⁻¹) :
3300, 2110 (CH=C-) ; 1765 (-OCOO-) ;
1610, 1510 (Phe) ;
1250 (-O-)
¹H-NMR, δ (ppm) :
2.42 (CH≡C-) ; 4.89 (≡C-CH₂-) ;
7.10, 7.85 (Phe)

### Example 11

### 4,4′-Bis(propargyloxycarbonyloxy)diphenylsulfide

IR (cm⁻¹) :
3300, 2110 (CH≡C-) ; 1760 (-OCOO-) ;
1610, 1510 (Phe)
¹H-NMR, δ (ppm) :
2.40 (CH≡C-) ; 4.87 (≡C-CH₂-) ;
7.10, 7.95 (Phe)

### Example 12

### 4,4′-Bis(propargyloxycarbonyloxy)phenyl benzoate

IR (cm⁻¹) :
3300, 2100 (CH≡C-) ;
1760 - 1720 (-OCOO-) ;
1600, 1500 (Phe)
¹H-NMR, δ (ppm) :
2.39 (CH≡C-) ; 4.72 (≡C-CH₂-) ;
7.20, 7.88 (Phe)

### Example 13

### 4,4′-Bis(propargyloxycarbonyloxy)azobenzene

IR (cm⁻¹) :
3300, 2100 (CH≡C-) ; 1760 (-OCOO-) ;
1600, 1510 (Phe)
¹H-NMR, δ (ppm) :
2.39 (CH≡C-) ; 4.72 (≡C-CH₂-) ;
7.20, 7.90 (Phe)

### Example 14

### 4,4′-Bis(propargyloxycarbonyloxy)azoxybenzene

IR (cm⁻¹) :
3300, 2100 (CH≡C-) ; 1760 (-OCOO-) ;
1610, 1510 (Phe)
¹H-NMR, δ (ppm) :
2.42 (CH≡C-) ; 4.88 (≡C-CH₂-) ;
7.10, 7.95 (Phe)

### Example 15

### N-(4-propargyloxycarbonyloxybenzylidene)-4-propargyloxycarbonyloxyaniline

IR (cm⁻¹) :
3300, 2100 (CH≡C-) ; 1760 (-OCOO-) ;
1610, 1510 (Phe)
¹H-NMR, δ (ppm) :
2.42 (CH≡C-) ; 4.85 (≡C-CH₂-) ;
7.10, 7.85 (Phe)

### Example 16

### Bis(3,3-dibromo-4-propargyloxycarbonyloxyphenyl)sulfone

IR (cm⁻¹) :
3300, 2200 (CH≡C-) ; 1760 (-OCOO-) ;
1610, 1510 (Phe)
¹H-NMR, δ (ppm) :
2.40 (CH≡C-) ; 4.78 (≡C-CH₂-) ;
7.20, 7.85 (Phe)

### Example 17

### Bis[4-(1,1-dimethyl-2-propinyl)oxycarbonyloxyphenyl]sulfone

IR (cm⁻¹) :
3300, 2100 (CH≡C-) ; 1738 (-OCOO-) ;
1600, 1500 (Phe) ;
1350, 1150 (-SO₂-)
¹H-NMR, δ (ppm) :
1.30 (-CH₃) ; 2.55 (CH≡C-) ;
4.86 (≡C-CH₂-) ; 7.30, 7.90 (Phe) .

### Example 18

Analogous to Example 1, diphenylsulfone-4,4′-bischloroformate was reacted with propargyl alcohol. The product was identified to be the same as the product of Example 3.

### Example 19

### 4,4′-Bis(propargyloxycarbonyloxy)biphenyl

A flask equipped with a stirrer, thermometer, nitrogen gas tube and reflux condenser was charged with 18.6 g of 4,4′-dihydroxybiphenyl and 23.0 g of trimethylamine in 50.0 g of methylene chloride. To the flask was added 26.0 g of propargyl chloroformate dropwise over one hour and the mixture was allowed to react for 6 hours at 30 °C. After the reaction, the reaction mixture was treated with methylene chloride-water mixture and the organic phase was separated followed by drying over magnesium sulfate overnight. After filtering, the filtrate was evaporated in a rotary evaporator. The resulting crystals were recrystallized to give the title compound at a yield of 93.6 % of theory.
IR (cm⁻¹) 3330, 2110, 1750, 1600, 1500
¹H-NMR (δ, ppm) 2.55 (CH≡C-),
4.86 (≡C-CH₂-), 7.30, 7.90 (Ph)

### Example 20

### 2,7-Bis(propargyloxycarbonyloxy)naphthalene

Analogous to Example 19, 2,7-dihydroxynaphthalene was reacted with propargyl chloroformate to produce the title compound. Yield was 83.6 % theory. m.p. 94-96 °C.
IR (cm⁻¹) 3330, 2100, 1755, 1610, 1510
¹H-NMR (δ, ppm) 2.42 (CH≡C-),
4.86 (≡C-CH₂-), 7.20, 8.10 (Ph)

Analogous to Example 19, the following compounds were synthesized.

### Example 21

### 1,8-Bis(propargyloxycarbonyoxy)anthracene

Yield, 88.3 %
NMR (cm⁻¹) 3300, 2100, 1760, 1610, 1510
¹H-NMR (δ, ppm) 2.42 (CH≡C-),
4.89 (≡C-CH₂-), 7.10, 8.20 (Ph)

### Example 22

### 3-Chloro-4,4′-bis(propargyloxycarbonyloxy)biphenyl

Yield, 85.2 %
IR (cm⁻¹) 3300, 2100, 1760, 1610, 1510
¹H-NMR (δ, ppm) 2.40 (CH≡C-),
4.87 (≡C-CH₂-), 7.10, 7.95 (Ph)

### Example 23

### 4-Propargyloxycarbonyloxybiphenyl

Yield, 90.2 %
IR (cm⁻¹) 3300, 2100, 1760, 1610, 1510
1H-NMR (δ, ppm) 2.2.40 (CH≡C-),
4.87 (≡C-CH₂-), 7.10, 7.95 (Ph)

### Example 24

### 1-Propargyloxycarbonyloxynaphthalene

Yield, 83.2 %
IR (cm⁻¹) 3300, 2100, 1760, 1610, 1510
¹H-NMR (δ, ppm) 2.42 (CH≡C-),
4.88 (≡C-CH₂-), 7.10, 7.95 (Ph)

### Example 25

### 3,3′-Dimethyl-4,4′-bis(propargyloxycarbonyloxy)biphenyl

Yield, 93.6 %
IR (cm⁻¹) 3300, 2110, 1750, 1600, 1500
¹H-NMR (δ, ppm) 2.55 (CH≡C-),
4.86 (≡C-CH₂-), 7.30, 7.90 (Ph)

### Example 27

Analogous to Example 19, one mole of biphenyl-4,4′-bischloroformate was reacted with two moles of propargyl alcohol. The product was identified to be the same as the product of Example 19 in the IR spectrometry and ¹H-NMR. Yield, 63.6 % of theory.

## Claims

1. A compound of the formula: 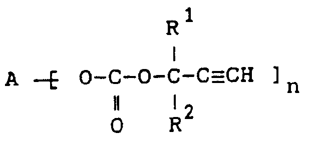 wherein:
A is an aromatic polycycle selected from the group consisting of a fused carbocycle having more than one benzene rings arranged in a straight row, a system having two benzene rings linked together with a direct bond, a C₁-C₂₀ alkylidene group, a cyclohexylidene group, -O-, -S-, -CO-, 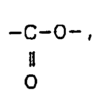 -CH=N-, -SO₂-, 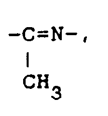 -N=N-, or 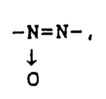 and derivatives thereof having on the benzene ring up to four substituent selected from the group consisting of a C₁-C₁₂ alkyl, a C₁-C₁₂ alkoxy, halo, nitro and cyano;
R¹ and R² are independently a hydrogen atom or C₁-C₁₂ alkyl; and
n is an integer of 1, 2 or 3.

2. The compound as claimed in Claim 1, wherein R¹ and R² are each hydrogen atom and n equals 2.

3. The compound as claimed in Claim 2, wherein said fused carbocycle is naphthalene.

4. The compound as claimed in Claim 2, wherein said fused carbocycle is anthracene.

5. The compound as claimed in Claim 2, wherein said system is biphenyl.

6. The compound as claimed in Claim 2, wherein said system is diphenylmethane.

7. The compound as claimed in Claim 2, wherein said system is 1,1-diphenylethane.

8. The compound as claimed in Claim 2, wherein said system is diphenyl ether.

9. The compound as claimed in Claim 2, wherein said system is diphenylsulfone.

10. The compound as claimed in Claim 2, wherein said system is benzophenone.

11. The compounds as claimed in Claim 1, which are
2,7-bis(propargyloxycarbonyloxy)naphthalene,
1,8-bis(propargyloxycarbonyloxy)anthracene,
4,4′-bis(propargyloxycarbonyloxy)biphenyl,
bis(4-propargyloxycarbonyloxyphenyl)methane,
1,1-bis(4-propargyloxycarbonyloxy)ethane,
4,4′-bis(propargyloxycarbonyloxy)diphenyl ether,
4,4′-bis(propargyloxycarbonyloxy)diphenylsulfone,
4,4′-bis(propargyloxycarbonyloxy)benzophenone,
4-propargyloxycarbonyloxybiphenyl, or
1-propargyloxycarbonyloxynaphthalene.

## Patentansprüche

1. Verbindung der Formel 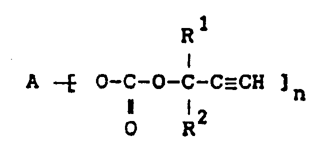 worin
A für ein aromatisches polyzyklisches System steht, das ausgewählt ist unter einem kondensierten carbozyklischen System mit mehr als einem Benzolring, die in gerader Linie aneinandergereiht sind, einem System mit zwei Benzolringen, die über eine Direktbindung miteinander verbunden sind, einer C₁-C₂₀-Alkylidengruppe, einer Cyclohexylidengruppe, -O-, -S-, -CO-, 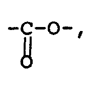 -CH=N-, -SO₂-, 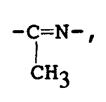 -N=N-, oder 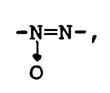 und Derivaten davon, die an dem Benzolring bis zu vier Substituenten besitzen, die ausgewählt sind unter C₁-C₁₂-Alkyl, C₁-C₁₂-Alkoxy, Halogen, Nitro und Cyano;
R¹ und R² unabhängig voneinander für ein Wasserstoffatom oder C₁-C₁₂-Alkyl stehen; und
n für 1, 2 oder 3 steht.

2. Verbindung gemäß Anspruch 1, worin R¹ und R² jeweils für ein Wasserstoffatom stehen und n für 2 steht.

3. Verbindung gemäß Anspruch 2, worin das kondensierte carbozyklische System Naphthalin ist.

4. Verbindung gemäß Anspruch 2, worin das kondensierte carbozyklische System Anthracen ist.

5. Verbindung gemäß Anspruch 2, worin das System Biphenyl ist.

6. Verbindung gemäß Anspruch 2, worin das System Diphenylmethan ist.

7. Verbindung gemäß Anspruch 2, worin das System 1,1-Diphenylethan ist.

8. Verbindung gemäß Anspruch 2, worin das System Diphenylether ist.

9. Verbindung gemäß Anspruch 2, worin das System Diphenylsulfon ist.

10. Verbindung gemäß Anspruch 2, worin das System Benzophenon ist.

11. Verbindungen gemäß Anspruch 1, nämlich 2,7-Bis(propargyloxycarbonyloxy)naphthalin, 1,8-Bis(propargyloxycarbonyloxy)anthracen, 4,4′-Bis(propargyloxycarbonyloxy)biphenyl, Bis(4-propargyloxycarbonyloxyphenyl)methan, 1,1-Bis(4-propargyloxycarbonyloxy)ethan, 4,4′-Bis(propargyloxycarbonyloxy)diphenylether, 4,4′-Bis(propargyloxycarbonyloxy)diphenylsulfon, 4,4′-Bis(propargyloxycarbonyloxy)benzophenon, 4-Propargyloxycarbonyloxybiphenyl oder 1-Propargyloxycarbonyloxynaphthalin.

## Revendications

1. Composé répondant à la formule : 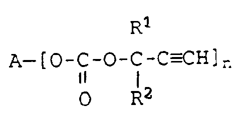 dans laquelle :
A est un composé polycyclique aromatique choisi parmi un composé carbocyclique condensé ayant plusieurs noyaux benzéniques formant une rangée linéaire, un système ayant deux noyaux benzéniques liés l'un à l'autre par une liaison directe, un groupe alkylidène en C₁-C₂₀, un groupe cyclohexylidène, -O-, -S-, -CO-, 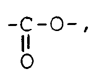 -CH=N-, -SO₂-, 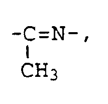 -N=N-, ou 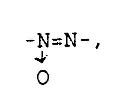 et des dérivés de ceux-ci ayant sur le noyau benzénique jusqu'à quatre substituants choisis parmi des groupes alkyle en C₁-C₁₂, alcoxy en C₁-C₁₂, halo, nitro et cyano ;
R¹ et R² sont indépendamment un atome d'hydrogène ou un groupe alkyle en C₁-C₁₂ ; et
n est un entier égal à 1, 2 ou 3.

2. Composé selon la revendication 1, dans lequel R¹ et R² sont chacun un atome d'hydrogène et n est égal à 2.

3. Composé selon la revendication 2, dans lequel ce composé carbocyclique condensé est le naphtalène.

4. Composé selon la revendication 2, dans lequel ce composé carbocyclique condensé est l'anthracène.

5. Composé selon la revendication 2, dans lequel ce système est le biphényle.

6. Composé selon la revendication 2, dans lequel ce système est le diphénylméthane.

7. Composé selon la revendication 2, dans lequel ce système est le 1,1-diphényléthane.

8. Composé selon la revendication 2, dans lequel ce système est l'éther diphénylique.

9. Composé selon la revendication 2, dans lequel ce système est la diphénylsulfone.

10. Composé selon la revendication 2, dans lequel ce système est la benzophénone.

11. Composés selon la revendication 1, qui sont :
le 2,7-bis(propargyloxycarbonyloxy)naphtalène,
le 1,8-bis(propargyloxycarbonyloxy)anthracène,
le 4,4′-bis(propargyloxycarbonyloxy)biphényle,
le bis(4-propargyloxycarbonyloxyphényl)méthane,
le 1,1-bis(4-propargyloxycarbonyloxy)éthane,
l'éther 4,4′-bis(propargyloxycarbonyloxy)diphénylique,
la 4,4′-bis(propargyloxycarbonyloxy)diphénylsulfone,
la 4,4′-bis(propargyloxycarbonyloxy)benzophénone,
le 4-propargyloxycarbonyloxybiphényle ou
le 1-propargyloxycarbonyloxynaphtalène.
